# EUROPEAN PATENT APPLICATION

(11) **EP 4 266 322 A1**
(43) Date of publication of application: **25.10.2023**
(21) Application number: 22170444.8
(22) Date of filing: 28.04.2022
(51) Int. Cl.: G16H 40/20, A61B 5/00, A61B 6/00, A61B 8/00, G06F 16/00, G06Q 10/00, G09B 5/00, G16H 40/67, G16H 50/70, G16H 70/20, G16H 80/00, G16H 10/60

(54) **SYSTEMS AND METHODS TO CONTEXTUALIZE CLINICAL PRODUCT/WORKFLOW ISSUES FOR STREAMLINED RESOLUTIONS/RECOMMENDATIONS**

(30) Priority: 19.04.2022 US 202263332294 P
(71) Applicant: Koninklijke Philips N.V., 5656 AG Eindhoven (NL)
(72) Inventor: STOLIKJ, Milosh, Eindhoven (NL); SHRUBSOLE, Paul Anthony, Eindhoven (NL); VAN DE WOUW, Doortje, Eindhoven (NL)
(74) Representative: Philips Intellectual Property & Standards

(57) **Abstract**

A recommender for recommending a resolution for an issue arising from a medical procedure operates as follows. The method includes mining, from one or more databases and responsive to a request for assistance, information related to one or more of a medical device used in the medical procedure and the medical professional performing the medical procedure. An assistance pathway resource is recommended from a plurality of assistance pathway resources for the medical professional based on the mined data. An indication of the recommended assistance pathway resource is output.

## Description

### FIELD OF THE INVENTION

The following relates generally to the medical imaging arts, medical imaging device maintenance arts, medical imaging device issue resolution arts, and related arts.

### BACKGROUND OF THE INVENTION

Medical professionals are frequently presented with clinical product issues that are difficult to resolve. Currently, medical professionals deal with issues by attempting to resolve the issue themselves by searching through online resources such as manufacturer-owned (e.g., technical documentation, learning materials), or public/third party owned resources (e.g., Internet search). Medical professionals can also ask an expert in their institution or department for assistance or call an outside party such as a clinical application specialist.

However, these current solutions can have drawbacks. For example, the current solutions can be time-consuming because the medical professional has difficulty in forming a search query for finding appropriate materials in product documentation/learning systems/online resources, has difficulty to express the situation of their problem to super users/clinical application specialists, or clinical application specialists may not be immediately available.

These drawbacks are particularly significant in time-constrained situations. For example, if a medical imaging technician encounters a problem while performing an imaging examination on a patient, the problem should preferably be resolved within a few minutes. Otherwise, the examination is delayed and the (typically tight) imaging examination schedule is adversely affected. Even worse, if the technician cannot solve the problem within a few tens of minutes then the imaging examination may need to be aborted and rescheduled, which incurs substantial monetary costs and inconvenience to the patient, as well as potentially delaying diagnosis and treatment of the patient. If the imaging technician attempts self-help and fails and only thereafter seeks assistance from a specialist, this delays the problem resolution. On the other hand, if the imaging technician initially consults a specialist for a problem that could have been resolved by self-help, then this also introduces costs in terms of the valuable time of the specialist.

The following discloses certain improvements to overcome these problems and others.

### SUMMARY OF THE INVENTION

In some embodiments disclosed herein, a non-transitory computer readable medium stores instructions executable by at least one electronic processor to perform a method of recommending a resolution for an issue arising from a medical procedure. The method includes: receiving a request for assistance with the issue from a medical professional performing the medical procedure; mining, from one or more databases, information related to one or more of a medical device used in the medical procedure and the medical professional performing the medical procedure; recommending an assistance pathway resource from a plurality of assistance pathway resources for the medical professional based on the mined data; and outputting, on a display device of an electronic processing device operable by the medical professional, an indication of the recommended assistance pathway resource.

In some embodiments disclosed herein, a medical apparatus is disclosed, including a medical device and an electronic processing device. The latter includes at least one electronic processor programmed to: receive a request for assistance with the issue from a medical professional performing the medical procedure; mine, from one or more databases, information related to one or more of a medical device used in the medical procedure and the medical professional performing the medical procedure; recommend an assistance pathway resource from a plurality of assistance pathway resources for the medical professional based on the mined data; and output, on a display device of an electronic processing device operable by the medical professional, an indication of the recommended assistance pathway resource.

In some embodiments disclosed herein, a method of recommending a resolution for an issue arising from a medical procedure is disclosed. The method includes: mining, from one or more databases and responsive to a request for assistance, information related to one or more of a medical device used in the medical procedure and the medical professional performing the medical procedure; recommending an assistance pathway resource from a plurality of assistance pathway resources for the medical professional based on the mined data; and outputting, on a display device of an electronic processing device operable by the medical professional, an indication of the recommended assistance pathway resource as a hyperlink to the recommended assistance pathway resource.

One advantage resides in providing relevant educational materials for a medical professional to resolve an issue with a medical procedure.

Another advantage resides in providing guided support for a medical professional based on their previous actions to resolve an issue with a medical procedure.

Another advantage resides in providing relevant materials for a medical professional to resolve an issue with a medical procedure without the use of a clinical application specialist.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically illustrates a medical device recommendation apparatus in accordance with the present disclosure.
Fig. 2 diagrammatically illustrates a medical device recommendation method using the apparatus of Fig. 1.

### DETAILED DESCRIPTION OF EMBODIMENTS

The following discloses an approach for providing context-based help when an imaging technician encounters a problem during an imaging session. In general, there are various avenues of assistance, such as: (1) self-help by referencing a manual or other documentation; (2) automated "wizard" help in which a computer-based system such as the scanner controller provides step-by-step instructions via a sequence of user dialog boxes for performing a task; (3) referral to a clinical application specialist (e.g. from a vendor of a medical imaging device); or (4) referral to an expert at the hospital or at another medical institution. Since the help is needed during an imaging examination, it is beneficial to have the help provided as soon as practicable.

The disclosed system mines data sources such as the technician's educational background (mined from the online training system); skill level (for example, assumed based on the technician's job title (including specializations, roles and competencies), and/or from the number of years employed, and/or obtained from electronic records of usage of the imaging system by the technician); the imaging system state (obtained from the imaging controller or ancillary equipment controllers, e.g. information such as the imaging modality and protocol, any failure codes, or so forth); and patient information (obtained from the radiology scheduler and electronic health records). In some embodiments, the technician requesting help may enter a natural language description of the problem and if so then this can be mined for further information about the context.

The disclosed system includes a recommender that recommends the help pathway (e.g., self-help, wizard, or clinical application specialist) based on similar historical help requests. Other inputs to the recommender may include the sources of available help, such as relevant manuals, relevant step-by-step wizards, and information on immediate clinical application specialist availability. Based on the recommender output, the imaging technician is directed to the appropriate type of help, preferably including providing information such as a link to the relevant manual or wizard, contact information for the specialist, and/or so forth.

In a variant embodiment, rather than using a clinical application specialist on staff with the vendor of the medical device, the system could provide inter-hospital assistance. In this variant embodiment, collaborating hospitals provide contact and schedule information for clinical application specialists employed by the various hospitals, and the system may then identify an immediately available clinical application specialist at the same hospital as the technician requesting help, or at another collaborating hospital. If the recommender determines this clinical application specialist is the appropriate avenue for help, then it places the imaging technician into contact with that clinical application specialist.

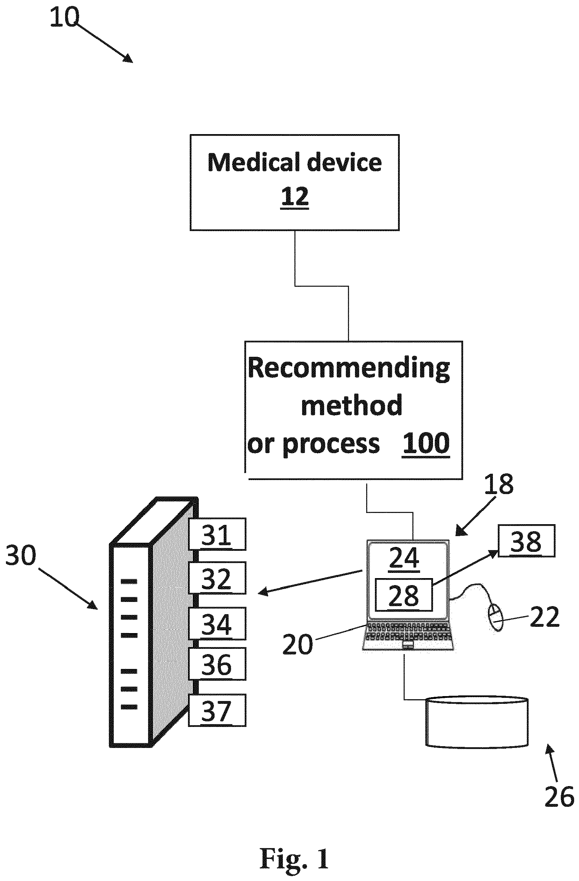

With reference to Fig. 1, an illustrative apparatus 10 for recommending a resolution for an issue arising from a medical procedure using a medical device 12 is shown. The medical device 12, for example, can comprise a medical imaging device 12 (also referred to as a medical device, an imaging device, imaging scanner, and variants thereof) can be a magnetic resonance imaging (MRI) scanner, a computed tomography (CT) scanner (as illustrated), a positron emission tomography (PET) scanner, a gamma camera for performing single photon emission computed tomography (SPECT), an interventional radiology (IR) device, an X-ray device, an image guided therapy (iGT) device, or so forth. Although shown in Fig. 1 as an imaging device (specifically a CT scanner), the medical device 12 can also be any other suitable medical device, such as a patient monitor, a radiation therapy device, a mechanical ventilator, and so forth, for which prompt assistance with a problem in operation of the medical device is desired.

An electronic processing device 18, such as a workstation computer, or more generally a computer, a smart device (e.g., a cellular telephone ("cell phone"), a smart tablet, and so forth), is operable by a medical professional (e.g., a doctor, nurse, technician, and so forth). The electronic processing device 18 may also include a server computer or a plurality of server computers, e.g., interconnected to form a server cluster, cloud computing resource, or so forth, to perform more complex computational tasks. The electronic processing device **18** includes typical components, such as an electronic processor **20** (e.g., a microprocessor), at least one user input device (e.g., a mouse, a keyboard, a trackball, and/or the like) **22**, and a display device **24** (e.g., an LCD display, plasma display, cathode ray tube display, and/or so forth). In some embodiments, the display device **24** can be a separate component from the electronic processing device **18**, or may include two or more display devices.

The electronic processor **20** is operatively connected with one or more non-transitory storage media **26.** The non-transitory storage media **26** may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation **18**, various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a visualization of a graphical user interface (GUI) **28** for display on the display device **24.**

The electronic processing device **18** is also in communication with a database **30** (shown in Fig. 1 as a server computer) that stores information related to the medical device **12** used in the medical procedure and/or the medical professional performing the medical procedure. The database **30** can also store historical help requests **31** received by the system in the past, along with information on the assistance pathway that resolved each such help request. The information can include, for example, a technician's educational data, a technician's skill level data including a number of successful procedures and how advanced those procedures were (e.g. if a user has a problem with a basic procedure, but they have succeeded in more advanced variants of that procedure, then it's likely to require an application specialist because this implies a machine related problem), a job title of the medical professional, a state of the medical device **12**, patient data for the patient undergoing the medical procedure, a technician's experience data relating to the number of times a particular procedure was recorded for that user. Skill relates to the and so forth. The database **30** can also store include service manuals **32** related to diagnostic procedures for a plurality of different medical devices (including the medical device **12** shown in Fig. 1). The database **30** also stores historical service records **34** related to previously-performed diagnostic procedures for diagnosing various malfunctions for a plurality of different medical devices (possibly including the medical device **12** shown in Fig. 1). The database **30** can further store online training materials or resources **36** related to the medical procedure, such as a medical procedure assistance program. The medical assistance research program could, for example, comprise an electronic processor programmed to execute one or more instructions for outputting a recommendation for an operation to be performed on the medical imaging device in order to resolve the issue arising from the medical procedure. Each of the service manuals **32**, historical service records **34**, and/or online training materials or resources **36**, along with the information related to the medical device **12** used in the medical procedure and/or the medical professional performing the medical procedure, can be stored in separate databases implemented in the server computer **30**, or stored on separate server computers. The database **30** can further store information **37** on experts at the hospital or at another medical institution that could be consulted to resolve a problem in operating the medical device **12.** This information **37** may include contact information, and may also optionally include links to electronic calendars of the experts in order to determine availability of a given expert.

The apparatus **10** is configured as described above to perform a method or process **100** of recommending a resolution for an issue arising from a medical procedure. The non-transitory storage medium **26** stores instructions which are readable and executable by the at least one electronic processor **20** to perform disclosed operations including performing the servicing method or process **100.** In some examples, the method **100** may be performed at least in part by cloud processing.

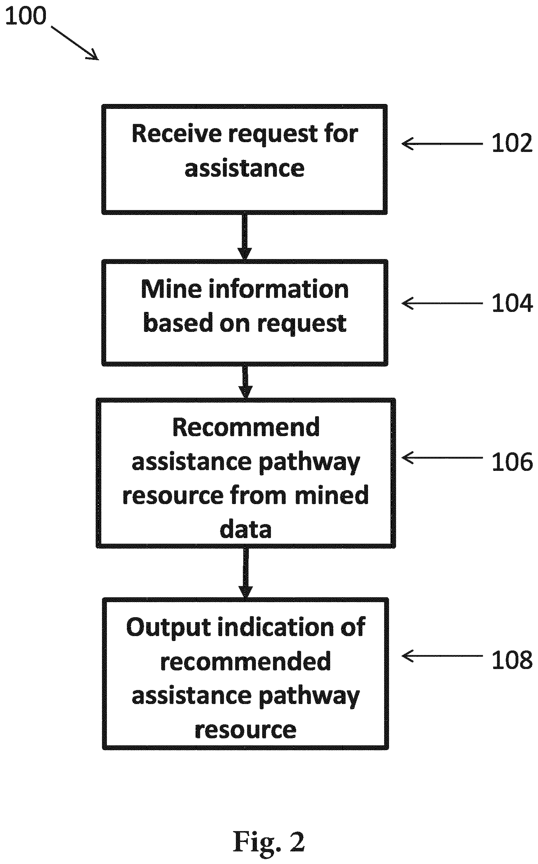

With reference to Fig. 2, and with continuing reference to Fig. 1, an illustrative embodiment of an instance of the method **100** is diagrammatically shown as a flowchart. At an operation **102**, a request for assistance with the issue with the medical procedure is received from the medical professional performing the medical procedure using the medical device **12** (i.e., a medical imaging device). Typically, the request for assistance is related to operation of the medical device **12**, and may be time-critical since, for example, the request for assistance may be made due to a real-time difficulty in using the medical device **12** to image or otherwise assess or treat a patient. By way of some non-limiting illustrative examples, the issue arising from the medical procedure could be a technical issue with the medical imaging device such as an image quality issue, a wired or wireless communication issue with ancillary equipment (for example, an MRI unable to properly communicate with a wired or wireless local MR receive coil) or other fault with the medical device **12.** In other embodiments, the issue arising from the medical procedure could be an operational issue such as the imaging technician having difficulty determining an optimal imaging parameter setting. In some examples, the request can include a text description of the request for assistance (note, the text description may in some embodiments be generated by automated speech-to-text conversion of a verbal description of the request provided by the medical professional). Moreover, while an imaging technician (or technician) is referred to in some illustrative examples, more generally this could be any medical professional who is making a request for assistance.

At an operation **104**, information related to one or more of the medical devices **12** used in the medical procedure and the medical professional performing the medical procedure can be mined from one or more databases (i.e., the database **30**). The mining operation **104** can include, for example, mining information including one or more of: a technician's (or more generally medical professional's) educational data, a technician's skill level data, a state of the medical device **12**, patient data, and so forth. When a text description of the request for assistance is provided, the mining operation **104** can include mining information from the text description of the request for assistance, for example using keyword extraction and/or natural language processing (NLP) techniques. In another example, the mining operation **104** includes retrieving a job title of the medical professional performing the medical procedure and mining information related to the medical professional performing the medical procedure from at least one of (i) an online training system used by the medical professional and (ii) a log of past medical procedures performed by the medical professional performing the medical procedure. In a further example, the mining operation **104** includes mining information related to the medical device **12** used in the medical procedure from an electronic controller (not shown) of the medical device **12.** These are merely examples, and should not be construed as limiting.

At an operation **106**, an assistance pathway resource from a plurality of assistance pathway resources is recommended for the medical professional based on the mined data. For example, the assistance pathway resources can include an online training resource related to the medical procedure, a medical procedure assistance program implemented on either the medical device **12** or the electronic processing device **18**; a referral to another medical professional, and so forth. The online training resource and/or the medical procedure assistance program can be stored in the database **30.** In some examples, when the text description of the request for assistance is provided, the recommending operation **106** can include recommending the assistance pathway resource further based on the information mined from the text description of the request for assistance. In another example, during the requesting operation **102**, the user can provide inputs related to each assistant pathway resource, and the mining operation **104** includes mining the information based on the inputs. The recommending operation **106** then includes recommending the appropriate assistance pathway resource based on the inputs. In one approach, the recommending operation **106** compares these inputs with the stored information **31** on historical help requests received by the system in the past. The most common successful assistance pathway for the most similar historical help requests (also stored as part of the information **31**) can then serve as the recommended assistance pathway. In another (not necessarily mutually exclusive) approach, if the information **37** on experts at the hospital or at another medical institution that could be consulted to resolve the problem include availability information retrieved from electronic calendars of the experts, then this can be used to recommend a particular expert who is immediately available to assist in resolving the problem. In another approach, the recommending option **106** can employ a rules-based algorithm that recommends the assistance pathway based on analysis of the inputs in accordance with the algorithm rules. These are merely some nonlimiting illustrative examples.

At an operation **108**, an indication **38** of the recommended assistance pathway resource is output, for example on the display device **24** of the electronic processing device **18.** For example, the indication **38** can be a hyperlink to the recommended assistance pathway resource (e.g., a link to the online training resource, a link to run a medical procedure assistance program, a link to a video calling service or texting service with another medical professional, and so forth). In some examples, when the recommended assistance pathway resource comprises a referral to another medical professional, contact and schedule information for a plurality of medical professionals can be received, an available medical professional can be identified. For example, a communication path can then be established between the available medical professional and the medical professional performing the medical procedure.

### EXAMPLE

The apparatus (or system) **10** can be configured to uses a description of the issue by the user, given in natural language, in combination with rich contextual information about their past experience, the learning trajectory of the user, and the current state of the operating device(s), to identify the best venue for support. Furthermore, in the case of self-help, the system **10** can select learning material from a database, which is relevant to their situation. To do so, the apparatus **10** is configured to perform the following operations: a user presses the "help" button on their phone or on the medical apparatus, activating a 'support system' application. The user may optionally enter a brief description of their problem, in natural language or they can select, for example, "let the system identify the problem for me". The system **10** recognizes the user in terms of their learning consumption history, experience level, and experience with the medical system **12** or clinical procedure being performed. The system **10** identifies the knowledge of the user and their experience from, for an example, a human resources system. The system **10** identifies the current state of the medical apparatus **12** the user has been recently interacting with. The system **10** identifies the current type of workflow and the current step in the user's clinical workflow to establish the context of the intended purpose of the user at that step. The system **10** retrieves available performance support systems. The system **10** retrieves available clinical experts. The system 10 retrieves list of available content for the user, from a content management system. The system 10 filters content based on the user's known gaps in knowledge and experience with the current state of the medical system to give immediate recommendations. The system **10** decides what type of support is best suitable for the user and provides the appropriate response.

To perform these operations, the electronic processor **20** of the electronic processing device **18** can include one or more corresponding modules specifically implemented for one or more of the operations. For example a module for selecting a support method can include a classification algorithm (e.g., the mining operation **104**). The classification is done based on the following input data: a problem description, entered by the user in natural language (the input is assumed to be in textual format, but can also be entered via speech interface, In the former case, the textual data is transformed into a vector format using an algorithm such as TF-IDF or using an embedding neural network. In the latter case, the speech can be converted first to text, via a speech-to-text algorithm, and is then converted to a vector format); a user profile, which is queried from an HR system (e.g., junior/senior), and the current career track (e.g., in training for resident); a device context of the medical device **12**, a user context a user knowledge, and so forth.

The result are three numerical values between [0, 1], each representing the confidence of the system for routing the request to self-help service/performance support service/human expert, respectively (i.e., 1 represents the maximum confidence).

In another example, multiple modules can be provided to determine support context (i.e., the recommending operation **106**). A first module can, given the user description of a problem, filter out collected data which is relevant to the given request. For example, if the user mentions the keywords 'MRI,' 'Compressed SENSE,' the first module can return machine log data relevant to the MRI exams requiring Compressed SENSE, such as whether it was used in the latest exams, which values were set, etc. The mapping from user description to features can be done by, for example, a look up table stored within the first module. For each mapping, the first module would also contain a set of instructions for which transformation to apply to the device log data to produce the required output. The output can be presented in aggregated form, indicating summary information regarding various aspects of the device function. An example output is provided as Table 1 below.

**Table 1**

| **Key** | **Value** | **Subkey** | **Value** |
|---|---|---|---|
| Feature | MRI Compressed SENSE | | |
| Parameter | Reduction Factor | Used Range | 0 (10%), 2 (20%), 5 (70%) |
| Parameter | Denoising factor | Used Range | Default (100%) |
| Potential issues | Image Quality | Confidence | 50% |

The first module also provides information about the state of the medical devices within a hospital. The state includes the following data: a list of activities performed at the medical device **12** ( e.g. an MRI exam, comprising multiple scans), an indication that the user has performed each activity; an output of each activity (i.e., a failure code), system settings for each activity (e.g. type of scan, scan parameters, etc.) and so forth.

A second module can, given the user description of a problem, filter out clinical data which is relevant to the given request. The data for this second module may originate from the hospital information system. This includes, for example, patient information, type of protocol used, involved health care professionals, etc. For example, given the keywords 'MRI', 'Compressed SENSE', the second module may return the protocols and anatomies which were scanned in the hospital. The results are again returned in aggregate format, indicating, for example, how frequently protocols and anatomies are run, and how frequently patients have been re-scanned.

In addition, to perform the recommending operation **106**, other modules can be included. For example, a third module can be implemented to determine the knowledge of the user regarding the used features of the medical device **12** to determine which aspects of the device **12** functionality/clinical aspects are relevant. For each aspect, the third module computes the proficiency of the user, based on the type and number of interactions the user has had with appropriate learning content, or based on a number of times the user has performed the particular activity without failure. Examples of data points that influence such calculation can include whether the user has following beginner/intermediate/advanced courses on a related topic, time elapsed since the last time a related learning material has been accessed, a test score on quizzes/exams on a related topi, and so forth.

A fourth module can be implemented to rank the relevance of different content material, based on the previous interactions of the user with the learning management system, the device and clinical context, and the current issue they are experiencing. An example implementation of the fourth module may be to perform a search in the learning management system, using the problem description as a query, and then to rank each search results with a score, given the user knowledge, device context and clinical context inputs. The scoring can be done based on manually crafted rules (i.e. a look up table), or using a machine learning algorithm (i.e., a learning to rank approach).

In some embodiments, the apparatus **10** can be used in conjunction with smart support tickets. To do so, the apparatus **10** automatically generates a machine and human readable support ticket immediately after the user presses the help button that can be routed and escalated throughout a cloudbased clinical workflow support system. The details of the ticket are articulated using the method described in the invention. The ticket is used, for example to gather statistics on types of encountered problems and how they were solved to improve the education and performance support content, or determine a ground truth (clinical context) when a problem has been escalated with an expert. The support tickets can be routed through a number of support channels via a remote command center which can provide remote support to multiple hospital sites simultaneously. The routing channels would start within the hospital (also to identified super users who are available in the current shift), then to the command center and then to a pool of available remote application specialists in the area or beyond. The characteristics for smart support tickets include the urgency and importance of the problem, which can be automatically deduced from schedule of the device (incurred delays and complexity of procedure). The support tickets can be pushed to a user interface on a number of different devices depending on the routing preferences. For example, they can be pushed to the mobile phone of a super-user within the hospital using the same platform as the performance support application.
The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium (**26**) storing instructions executable by at least one electronic processor (**20**) to perform a method (**100**) of recommending a resolution for an issue arising from a medical procedure, the method including:
receiving a request for assistance with the issue from a medical professional performing the medical procedure;
mining, from one or more databases (**30**), information related to one or more of a medical device (12) used in the medical procedure and the medical professional performing the medical procedure;
recommending an assistance pathway resource from a plurality of assistance pathway resources for the medical professional based on the mined data; and
outputting, on a display device (**24**) of an electronic processing device (**18**) operable by the medical professional, an indication (**38**) of the recommended assistance pathway resource.

2. The non-transitory computer readable medium (**26**) of claim 1, wherein the medical device (12) is a medical imaging device, and the issue arising from the medical procedure comprises a fault with the medical imaging device.

3. The non-transitory computer readable medium (**26**) of either one of claims 1 and 2, wherein the method (**100**) further includes:
receiving, via at least one user input device (**24**) of the electronic processing device (**18**), inputs related to each assistance pathway resource; and
mining the information from the one or more databases (**30**) based on the received inputs.

4. The non-transitory computer readable medium (**26**) of any one of claims 1-3, wherein the method (**100**) further includes:
receiving contact and schedule information for a plurality of medical professionals; and
identifying, from the recommended assistance pathway resource, an available medical professional.

5. The non-transitory computer readable medium (**26**) of claim 4, wherein the method (**100**) further includes:
establishing a communication path between the available medical professional and the medical professional performing the medical procedure.

6. The non-transitory computer readable medium (**26**) of any one of claims 1-5, wherein the plurality of assistance pathway resources comprise:
an online training resource related to the medical procedure;
a medical procedure assistance program implemented on either the medical device (**12**) or the electronic processing device (**18**); and
a referral to another medical professional.

7. The non-transitory computer readable medium (**26**) of any one of claims 1-6, wherein mining, from one or more databases (**30**), information related one or more of a medical device (**12**) used in the medical procedure and a medical professional performing the medical procedure includes:
mining information including one or more of: a technician's educational data, a technician's skill level data, a technician's experience data, a state of the medical device, and patient data.

8. The non-transitory computer readable medium (26) of any one of claims 1-7, wherein:
the receiving of the request includes receiving a text description of the request for assistance;
the mining further includes mining information from the text description of the request for assistance; and
the recommending of the assistance pathway resource is further based on the information mined from the text description of the request for assistance.

9. The non-transitory computer readable medium (**26**) of any one of claims 1-8, wherein the mining includes mining information related to the medical device (**12**) used in the medical procedure from an electronic controller of the medical device.

10. The non-transitory computer readable medium (**26**) of any one of claims 1-9, wherein the mining includes retrieving a job title of the medical professional performing the medical procedure and mining information related to the medical professional performing the medical procedure from at least one of (i) an online training system used by the medical professional and (ii) a log of past medical procedures performed by the medical professional performing the medical procedure.

11. The non-transitory computer readable medium (**26**) of any one of claims 1-10, wherein the medical device (**12**) comprises a medical imaging device.

12. A medical apparatus (**10**), comprising:
a medical device (**12**); and
an electronic processing device (**18**) comprising at least one electronic processor (**20**) programmed to:
receive a request for assistance with the issue from a medical professional performing the medical procedure;
mine, from one or more databases (**30**), information related to one or more of a medical device used in the medical procedure and the medical professional performing the medical procedure;
recommend an assistance pathway resource from a plurality of assistance pathway resources for the medical professional based on the mined data; and
output, on a display device (**24**) of an electronic processing device (**18**) operable by the medical professional, an indication (**38**) of the recommended assistance pathway resource.

13. The apparatus (**10**) of either claim 12, wherein the at least one electronic processor (**20**) is programmed to:
outputting the indication (**38**) as a hyperlink to the recommended assistance pathway resource.

14. The apparatus (**10**) of either one of claims 12 and 13, wherein the at least one electronic processor (**20**) is programmed to:
receive contact and schedule information for a plurality of medical professionals;
identify, from the recommended assistance pathway source, an available medical professional; and
establish a communication path between the available medical professional and the medical professional performing the medical procedure.

15. The apparatus (**10**) of any one of claims 12-14, wherein the assistance pathway resources comprise:
an online training resource related to the medical procedure;
a medical procedure assistance program implemented on either the medical device (12) or the electronic processing device (**18**); and
a referral to another medical professional.
